# EUROPEAN PATENT APPLICATION

(11) **EP 2 436 271 A1**
(43) Date of publication of application: **04.04.2012**
(21) Application number: 11755939.3
(22) Date of filing: 18.03.2011
(51) Int. Cl.: A23L 1/00

(54) **EDIBLE FILM**

(30) Priority: 19.03.2010 JP 2010065243
(71) Applicant: Tsukioka Co., Ltd., Gifu 504-0925 (JP)
(72) Inventor: NISHIMURA, Misao, Kakamigahara-shi Gifu 504-0925 (JP); TSUKIOKA, Tadao, Kakamigahara-shi Gifu 504-0925 (JP)
(74) Representative: Berryman, Natalia Grace
(86) International application number: PCT/JP2011/001623
(87) International publication number: WO 2011/114749

(57) **Abstract**

An object of the invention is to provide an edible film which, even if accidentally swallowed, does not cause respiratory distress.

An edible film 1 has a predetermined planar shape and is adapted for use by dissolving in the mouth of a user. The edible film 1 is rectangular in shape and has a layered construction composed of two layers. The edible film 1 of this embodiment is **characterized by** having an opening-forming area 30. Although the film 1 does not have an opening formed therein prior to use, the opening-forming area is capable of forming an opening 40 of a predetermined shape that passes through the film from one side to another side thereof at the time of use.

## Description

### TECHNICAL FIELD

The present invention relates to an edible film, and more specifically to an edible film which, even if accidentally swallowed, does not pose a danger of suffocation.

### BACKGROUND ART

A number of edible films of a type used by mixing together various additives, such as algefacients and medicinal agents, and gradually having the film dissolve within the user's mouth so as to release the additives within the mouth have been developed (see, for example, Patent Documents 1 and 2). Such edible films are generally used by being attached within the oral cavity, such as on top of the tongue. Such a film, once it has been appropriately attached to the top of the tongue at the time of use, is dissolved by saliva and then can be swallowed, enabling the film to be used without obstructing the throat. However, with the edible films that have hitherto been disclosed, there is a possibility, albeit rare, that the film, instead of sticking to the top of the tongue, will stick directly to the throat, whereby a user will have difficulty to breath. Hence, there has existed a desire for the development of an edible film which, even in the event of accidental swallow, will not precipitate respiratory distress.

Patent Document 1: Japanese Patent Application Laid-open No. 2000-342193
Patent Document 2: Japanese Patent No. 3730081

### DISCLOSURE OF THE INVENTION

Accordingly, an object of the invention is to provide an edible film which, even if accidentally swallowed, does not cause respiratory distress.

As a result of extensive investigations, the inventors of the present invention have discovered that mere formation of holes in an edible film in order to allow the user's breath to pass through when the film has been accidentally swallowed causes a sensation that the film user finds unpleasant. With further investigations, the inventors have also found that the above object can be achieved by developing an edible film provided with an area which, if required, forms an opening at the time of use and arrived at the present invention. These findings ultimately led to the inventions which are recited below.
1. An edible film to be adopted for use by dissolving in the mouth of a user,
   comprising an opening-forming area in which an opening is not formed prior to use but an opening of a predetermined shape that passes through the film from one side to another side thereof can be formed at the time of use.
2. The edible film according to 1, wherein the opening-forming area is formed by a U-shaped incision.
3. The edible film according to 1, wherein the opening-forming area is formed by arranging perforations in a circular shape.
4. The edible film according to any of 1 to 3, wherein the opening-forming area is formed to form an opening having a diameter of from 3 mm to 10 mm.
5. The edible film according to claim 1, wherein the opening-forming area is formed by using a cutter heated to a temperature of between 0 and 150°C in order to make an incision in the film.
6. The edible film according to claim 1, wherein the edible film includes a water-soluble base.
7. An edible film to be adopted for use by dissolving in the mouth of a user,
   comprising film-splitting areas wherein the edible film is in a single film form prior to use and the film-spllitting areas enable the edible film to be separated into a plurality of edible film pieces at the time of use. (Hereinafter, inventions cited as a second invention correspond to this invention.)
8. The edible film according to 7, wherein the film-splitting areas are formed by arranging a plurality of rectilinear perforations.
9. The edible film according to 7, wherein the film-splitting areas are formed by arranging a plurality of curvilinear perforations.

The edible film of the invention, even if accidentally swallowed, will not cause respiratory distress in the user.

### BRIEF DESCRIPTION OF THE DRAWINGS

FIG. 1 is a perspective view showing a first embodiment of the edible film of the invention.
FIG. 2 is a perspective view showing the manner in which the edible film shown in FIG. 1 is used.
FIG. 3 is a perspective view showing another embodiment of the edible film of the invention.
FIG. 4 is a perspective view showing yet another embodiment of the edible film of the invention.
FIG. 5 is a perspective view showing a further embodiment of the edible film of the invention.
FIG. 6 is a perspective view showing a still further embodiment of the edible film of the invention.
FIG. 7 is a perspective view showing an additional embodiment of the edible film of the invention.
FIG. 8 is a perspective view showing a further embodiment of the edible film of the invention.
FIG. 9 is a perspective view showing a yet further embodiment of the edible film of the invention.

### EXPLANATION OF REFERENCE NUMERALS

- 1: edible film
- 10: first layer
- 20: second layer
- 30: opening-forming area
- 31: incision
- 40: opening

### BEST MODE FOR CARRYING OUT THE INVENTION

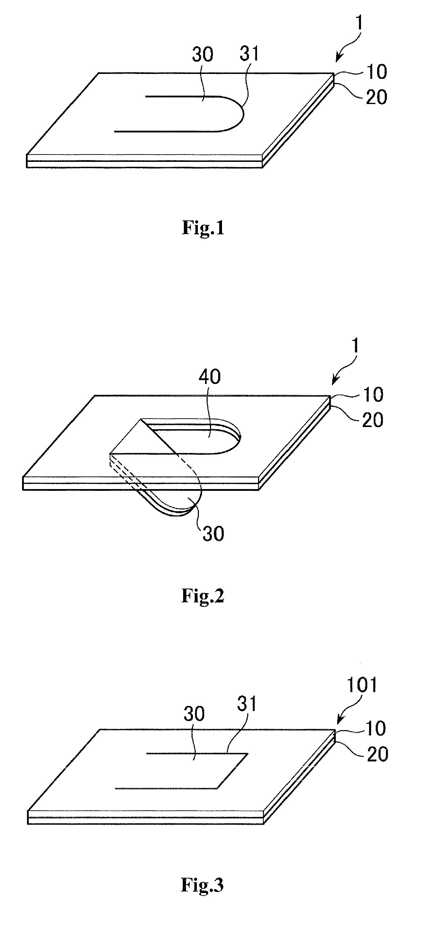

The edible film of the invention is described in detail below while referring to the diagrams. An edible film 1 according to the embodiment shown in FIG. 1 has a predetermined planar shape and is adapted for use by dissolving in the mouth of a user. The edible film 1 of this embodiment is rectangular in shape and has a layered construction composed of two layers. These features do not differ from those of conventional known edible films, in particular the film 1 has a first layer 10 and a second layer 20 placed on the first layer 10, with the first layer 10 and the second layer 20 each containing a water-soluble base and an additive.

No particular limitation is imposed on the materials which form the edible film of the invention. Provided they are materials which generally make up this type of edible film, use may be made of any water-soluble base and additives without particular limitation. Some examples are described below. The water-soluble base that is preferably used in the respective layers will differ according to the layered structure and intended use of the edible film, although use may generally be made of the following film-forming materials: polyvinyl pyrrolidone, polyvinyl alcohol, sodium polyacrylate, carboxymethyl cellulose, starch, xantham gum, karaya gum, sodium alginate, methyl cellulose, carboxyvinyl polymer, agar, hydroxypropyl cellulose, hydroxypropyl methylcellulose phthalate (HPMCP), cellulose acetate phthalate (CAP), carboxymethyl ethyl cellulose (CMEC), ethyl cellulose, hydroxyethyl cellulose, hydroxypropyl methyl cellulose, carboxyvinyl polymer (available from BF Goodrich under the trade name Carbopol), tragacanth, gum arabic, locust bean gum, guar gum, carrageenan, dextrin, dextran, amylose, carboxymethylcellulose potassium, carboxymethylcellulose sodium, carboxymethylcellulose calcium, pullulan, chitosan, starch, polyvinyl alcohol, carboxymethyl ethyl cellulose, carboxymethyl starch sodium, psyllium husk, galactomannan, Eudragit, casein, sodium alginate, alkyl esters of alginic acid, gelatin, shellac type resins (shellac, clear white shellac), starch, cellulose acetate, hydroxyethyl methyl cellulose, water-insoluble methacrylic acid copolymers, ethyl methacrylate-trimethylammonium ethyl copolymer, (dimethylamino)ethyl methacrylate-methyl methacrylate copolymer, pullulan, hydroxypropyl methyl cellulose (HPMC), methyl cellulose, acrylic acid, methyl methacrylate copolymer, hydroxyethyl cellulose and polyethylene glycol.

The additives that are preferably used will differ according to the layered structure and intended use of the edible film, although use may generally be made of the following medicinal ingredients: commonly used bases such as chitosan, starch and pectin; binders such as tragacanth powder, gum arabic and cornstarch; excipients such as crystalline cellulose; disintegrants such as cornstarch and gelatinized starch; lubricants such as magnesium stearate; sweeteners such as sucrose, lactose, fructose, saccharin and asparatame; flavoring agents such as peppermint, peppermint oil, cherry flavor, orange oil and fennel oil; preservatives such as benzoic acid, sodium benzoate, benzyl benzoate, ethyl p-oxybenzoate, butyl p-oxybenzoate and propyl p-oxybenzoate; opacifying agents such as titanium oxide; and colorants such as
iron sesquioxide and yellow iron sesquioxide. The amounts in which the additives are included may be set in accordance with the types of additives used and the combination with the water-soluble base, and are not subject to any particular limitation in the invention.

The edible film 1 of this embodiment is characterized by having an opening-forming area 30. Although the film 1 does not have an opening formed therein prior to use, the opening-forming area is capable of forming an opening 40 of a predetermined shape that passes through the film from one side to another side thereof (see FIG. 2) at the time of use. The opening-forming area 30 is formed by a U-shaped incision 31.

The edible film 1 of this embodiment has a rectangular shape, the size being preferably from 5 mm to 50 mm × from 5 mm to 50 mm. At a preferably total film thickness of from 50 µm to 5,000 µm, even when the film is attached to the upper jaw, it will not separate, and the sense of discomfort in the oral cavity will be minimal. The total thickness of the film is more preferably from 50 µm to 500 µm, and even more preferably from 50 µm to 100 µm. The thickness of the first layer is preferably from 20 µm to 300 µm, and more preferably from 20 µm to 100 µm; the thickness of the second layer is preferably from 3 µm to 300 µm, and more preferably from 3 µm to 30 µm. As for the size of the U-shaped incision, from the standpoint of securing the airway, it is preferable to make an incision such that the opening which forms when the incision opens up becomes an opening having a diameter of from 3 mm to 10 mm.

The edible film of this embodiment can be obtained by preparing a first layer-forming solution and a second layer-forming solution, casting each of the resulting solutions in order to form liquid films, drying the resulting liquid films to form an elongated film, cutting the elongated film at a predetermined position to give a rectangular shape, and using a U-shaped cutter to make a U-shaped incision at a predetermined position on the rectangular film. It is preferable to set the thickness of the cutter used at this time to from 0.2 mm to 1 mm, the cutter temperature when making the incision at between 0 and 150°C, and more preferably between 50 and 150°C, and the cutter height to from 3 mm to 18 mm. Any cutter that is commonly used to cut films may be used without particular limitation, although the use of a Teflon®-coated cutter is preferred because, with such a cutter, a good opening forms without fusion of the film material occurring near the incision.

Illustrative examples of solvents which may be used in the above first layer and second layer-forming solutions include water, ethanol, acetic acid, acetone, anisole, 1-butanol, 2-butanol, n-butyl acetate, t-butyl methyl ether, cumene, dimethylsulfoxide, ethyl acetate, diethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, tetrahydrofuran, acetonitrile, chlorobenzene, chloroform, cyclohexane, 1,2-dichloroethene, dichloromethane, 1,2-dimethoxyethane, N,N-dimethylacetamide, N,N-dimethylformamide, 1,4-dioxane, 2-ethoxyethanol, ethylene glycol, formamide, hexane, methanol, 2-methoxyethanol, methyl butyl ketone, methyl cyclohexane, N-methylpyrrolidone, nitromethane, pyridine, sulfolane, tetralin, toluene, 1,1,2-trichloroethene, xylene, 1,1-diethoxypropane, 1,1-dimethoxymethane, 2,2-dimethoxypropane, isooctane, isopropyl ether, methyl isopropyl ketone, methyl tetrahydrofuran, petroleum ether, trichloroacetic acid, trifluoroacetic acid, and methylene chloride. At the time of use, these may be used singly or as mixtures of two or more thereof. Of these solvents, the use of ethanol, water, ethyl acetate or a combination of these solvents (e.g., ethanol-water mixtures, ethanol-ethyl acetate mixtures) is most preferred.

The edible film 1 of this embodiment can be used by, for example, attaching the first layer to the upper jaw and having the layers dissolve into the oral cavity in order from the second layer, thereby enabling a time difference to be imparted to the effects of medicinal agents and supplements or food ingredients. Moreover, in the event that the film should be accidentally swallowed, as shown in FIG. 2, the U-shaped incision is pushed open in the direction of the arrow under pressure due to aspiration as the user attempts to breath, forming an opening 40. As a result, the airway is secured and the user does not suffer respiratory distress.

Next, other embodiments of the edible film of the invention are described. The following embodiments are described with particular emphasis on those features which differ from the embodiment illustrated above in FIGS. 1 and 2. To any features that are not described in detail, the abovementioned explanations apply. For example, in each of the following embodiments, an incision is formed so as to create an opening having a diameter of from 3 mm to 10 mm.

In the edible film 101 according to another embodiment shown in FIG. 3, the opening-forming area 30 is formed as a square U-shaped incision 32.

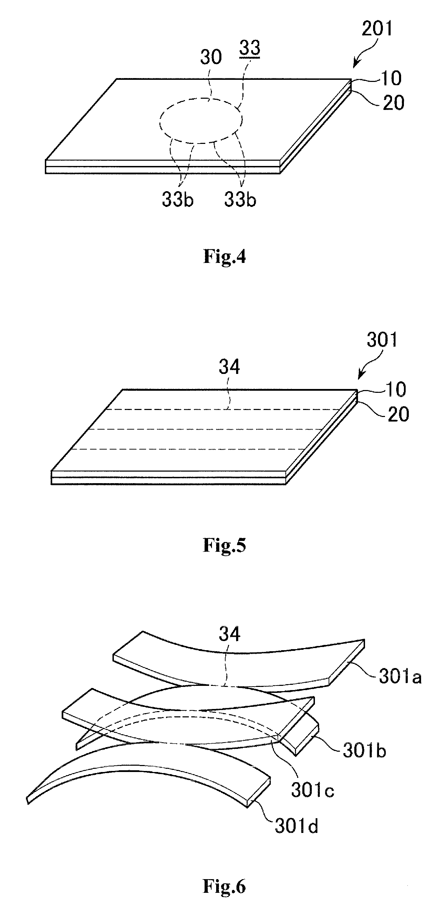

In the edible film 201 according to another embodiment shown in FIG. 4, the opening-forming area 30 is formed by arranging perforations 33 in a circular shape. Setting the length ratio of incised areas 33a to non-incised areas 33b in the perforations 33 to preferably from 5:1 to 20:1 is preferable for good formation of an opening.

Next, an embodiment according to the second invention is described while referring to FIGS. 5 and 6.
The edible film 301 of this embodiment, as shown in FIG. 5, is an edible film adapted for use by dissolving in the mouth of a user, the film having film-splitting areas 34. The edible film 301 is in the form of a single sheet of film prior to use. And the film-splitting areas enable the edible film to be separated into strips (see FIG. 6) as a plurality of edible film pieces at the time of use.
Specifically, the film-splitting areas 34 are formed by a plurality of rectilinear perforations.
Moreover, at the time of use, as shown in FIG. 6, even should the film separate from the mucosa of the oral cavity under the action of the user's saliva or the like, because the film splits at each film-splitting area 34 into a plurality of strips 301a, 301b, 301c and 301d, the film does not cram the throat and obstruct the airway.
Setting the length ratio of incised areas to non-incised areas at the perforations to from 5:1 to 20:1 is preferable for good splitting of the film.

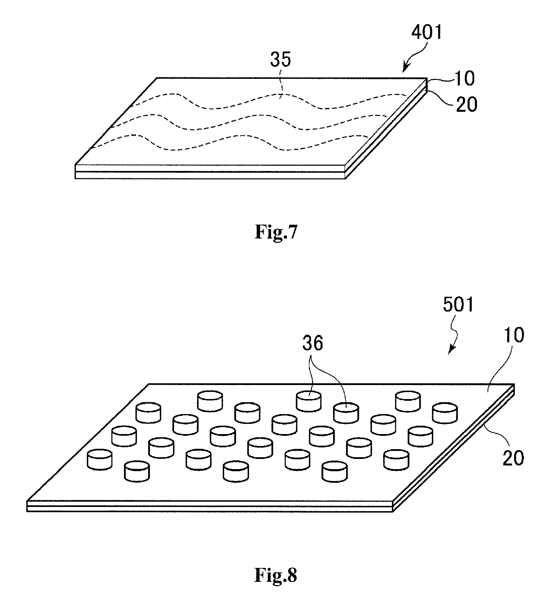

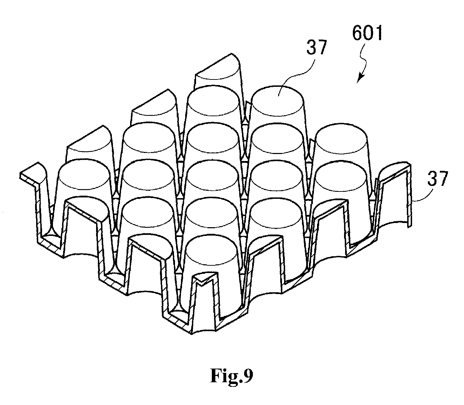

Next, another embodiment of the second invention is described while referring to FIG. 7.
In the edible film 401 of this embodiment, the film-splitting areas 35 are formed by a plurality of curvilinear perforations. This point differs from the embodiment shown in FIG. 5, aside from which the film-splitting areas 35 is formed in the same way.
Next, other embodiments of the edible film are described while referring to FIGS. 8 to 10.
In the edible film 501 of the embodiment shown in FIG. 8, a plurality of protrusions 36 is formed on one side of the film. The individual protrusions in this embodiment have circular shapes, but are not limited in this respect, various other forms, including rectangular or wavelike forms, being possible.
With regard to the size of the protrusions, the protrusions have a diameter of preferably from 2 to 5 mm, and the collective surface area over which the protrusions are provided preferably represents from 20 to 50% of the total surface area of the film.
The edible film of this embodiment may be obtained by producing the edible film in accordance with a conventional method, then subjecting the resulting film to embossing treatment.
Alternatively, in the edible film 601 of the embodiment shown in FIG. 9, raised and recessed areas 37 are formed in the film. The individual raised and recessed areas are of a size which is the same as the size of the protrusions in the embodiments shown in FIG. 8. In FIG. 9, although the layer structure of the film is omitted, as with the edible film shown in FIG. 8, the film is given a two-layer structure.
The edible film of this embodiment may be obtained by using a male-female embossing die to emboss the film.
By providing raised and recessed areas or protrusions in the manner of the edible films of the embodiments shown in FIGS. 8 and 9, even in cases where the user has begun to swallow the edible film before it is wetted with saliva, the airway can be secured, making it possible to prevent respiratory distress.

The present invention is not limited to the embodiments described herein, and may be practiced using various modifications and changes thereto insofar as they do not depart from the spirit and scope of the invention. For instance, although the edible films of these embodiments have been illustrated above by examples in which the films have a rectangular shape, the edible film of the invention is not limited to such examples, and may have various other shapes, such as square, circular or elliptical shapes. The edible films have also been illustrated by examples in which the films are composed of two layers, although a one-layer construction may also be effective.
The film thickness of 50 µm to 1,000 µm is not limited by the example above, and may be set to from 50 µm to 5,000 µm.
In the above examples, the number of film-splitting areas in the second aspect of the invention was set to three so that the film divides into four pieces, although the number of filmsplitting areas may be set as desired without being so limited. Also, in the above embodiment, the edible film pieces are described as being in the shape of strips, although they may alternatively be given various other shapes, such as square shapes, circular shapes or indefinite shapes. Moreover, in the foregoing examples, only cases in which perforations have been formed are presented, although the edible film is not limited in this respect, desirable effects being achievable even with socalled half-cuts obtained by cutting about halfway through the film in the thickness direction.

### EXAMPLES

The invention is illustrated more fully below by way of working examples and comparative examples, although these examples are not intended to limit the invention.

### Example 1

An edible film of the composition indicated below and having only a single-layer construction was prepared. In preparing the film, 45 parts by weight of the film-forming material of the following composition was dissolved in 55 parts by weight of water-containing alcohol (10 parts by weight) as the solvent, giving a film-forming solution. The film-forming solution was then cast to form a liquid film, and the liquid film was dried, giving an elongated film. This elongated film was cut at a predetermined position into a rectangular film, and a U-shaped incision (length, 15 mm; width, 7 mm) was made at a predetermined position in the rectangular film using a U-shaped Teflon®-coated cutter (thickness, 0.7 mm; cutter temperature, 70°C), thereby giving an edible film of the form shown in FIG. 1. The film thus obtained was of a rectangular shape 20 × 30 mm in size and had a weight of 43 mg/strip, a film thickness of 52 µm, and a caffeine content of 10 mg/strip.

### Edible Film Composition

| | |
|---|---|
| Water-soluble base (pullulan) | 37.9 parts by weight |
| Water-soluble base (starch) | 12.0 parts by weight |
| Additive (acetaminophen as medicinal agent) | 23.3 parts by weight |
| Additive (polyethylene glycol as plasticizer) | 20.0 parts by weight |
| Additive (polyoxyethylene sorbitan oleate as emulsifying agent) | 1.0 part by weight |
| Additive (powder reduced malt syrup as sweetener) | 5.5 parts by weight |
| Additive (sucralose as sweetener) | 0.2 part by weight |
| Additive (L-menthol as flavoring) | 0.1 part by weight |

The resulting edible films were wetted with water then placed so as to obstruct the opening of a 15 mm diameter glass funnel and aspirated, whereupon the U-shaped incision opened in the manner shown in FIG. 2 to create a flap, thus forming an opening that passed through the film.

## Claims

1. An edible film to be adopted for use by dissolving in the mouth of a user,
comprising an opening-forming area in which an opening is not formed prior to use but an opening of a predetermined shape that passes through the film from one side to another side thereof can be formed at the time of use.

2. The edible film according to claim 1, wherein the opening-forming area is formed by a U-shaped incision.

3. The edible film according to claim 1, wherein the opening-forming area is formed by arranging perforations in a circular shape.

4. The edible film according to any of claims 1 to 3, wherein the opening-forming area is formed to form an opening having a diameter of from 3 mm to 10 mm.

5. The edible film according to claim 1, wherein the opening-forming area is formed by using a cutter heated to a temperature of between 50 and 150°C in order to make an incision in the film.

6. The edible film according to claim 1, wherein the edible film includes a water-soluble base.

7. An edible film to be adopted for use by dissolving in the mouth of a user,
comprising film-splitting areas, wherein the edible film is in a single film form prior to use and the film-splitting areas enable the edible film to be separated into a plurality of edible film pieces at the time of use.

8. The edible film according to claim 7, wherein the film-splitting areas are formed by arranging a plurality of rectilinear perforations.

9. The edible film according to claim 7, wherein the film-splitting areas are formed by arranging a plurality of curvilinear perforations.
